# FASCICULE DE BREVET EUROPEEN

(11) **EP 3 501 608 B1**
(45) Date de publication et mention de la délivrance du brevet: **24.06.2020**
(21) Numéro de dépôt: 17306870.1
(22) Date de dépôt: 21.12.2017
(51) Int. Cl.: A61Q 19/02, A61K 8/9789

(54) **EXTRAIT ALCOOLIQUE DE PARTIES AÉRIENNES D'ANTHYLLIS VULNERARIA, SON PROCÉDÉ D'OBTENTION, ET COMPOSITION COSMÉTIQUE OU DERMATOLOGIQUE LE CONTENANT**
ALKOHOLISCHER AUSZUG AUS DEN OBERIRDISCHEN TEILEN VON ANTHYLLIS VULNERARIA, SEIN HERSTELLUNGSVERFAHREN UND KOSMETISCHE ODER DERMATOLOGISCHE ZUSAMMENSETZUNG, DIE IHN ENTHÄLT
ALCOHOLIC EXTRACT OF AERIAL PARTS OF ANTHYLLIS VULNERARIA, METHOD FOR OBTAINING SAME, AND COSMETIC OR DERMATOLOGICAL COMPOSITION CONTAINING SAME

(43) Date de publication de la demande: 26.06.2019
(73) Titulaire: Chanel Parfums Beauté, 92200 Neuilly-sur-Seine (FR)
(72) Inventeur: TORIBIO, Alix, 93400 SAINT OUEN (FR); WEINBERG, Lionel, 94170 LE PERREUX-SUR-MARNE (FR); LERISSON, Annie, 95360 MONTMAGNY (FR); GILLET, Maeva, 75012 PARIS (FR); BOUISSOU-CADIO, Emmanuelle, 93390 CLICHY SOUS BOIS (FR); LEJEUNE, François, 60570 ANDEVILLE (FR)
(74) Mandataire: Plasseraud IP

(56) Documents cités:
- FR-A1- 2 699 073
- FR-A1- 2 831 444
- FR-A1- 2 834 211
- FR-A1- 2 837 702
- HIRANO A: "Skin external preparation for use as cosmetics, comprises extracts of e.g. Aeschynomene indica, Alysicarpus vaginalis, Anthyllis vuleneraria, Butea monosperma, Judas tree and Campylotropis trigonoclada", WPI / THOMSON, 28 septembre 2001 (2001-09-28), XP002415823,
- "Gesture of Beauty Kit", GNPD, MINTEL, janvier 2015 (2015-01), XP002765376,
- "Natural Plus Sun Stick SPF", GNPD, MINTEL, juin 2017 (2017-06), XP002777039,
- "After sun lotion", GNPD; MINTEL, septembre 2008 (2008-09), XP002692716, [extrait le 2008-09-01]

## Description

L'invention concerne un extrait de parties aériennes d'*Anthyllis vulneraria,* son procédé d'obtention, une composition cosmétique ou dermatologique le contenant, ainsi que différentes utilisations cosmétiques.

La couleur de la peau humaine est fonction de différents facteurs et notamment des saisons de l'année, de la race et du sexe. Elle est principalement déterminée par la nature et la concentration de mélanine produite par les mélanocytes. Les mélanocytes sont les cellules dendritiques spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestroprogestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc), à défaut de pouvoir repigmenter la peau lésée, on achève de dépigmenter les zones de peau normale résiduelle pour donner à l'ensemble de la peau une teinte blanche homogène.

Une substance est reconnue comme dépigmentante ou anti-pigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

On a récemment établi un lien entre pigmentation et la famille de gènes des Sestrines (SESN) - notamment les SESN1 et SESN2. En effet, comme établi par la demanderesse dans sa demande EP2015/072080, leurs niveaux d'expression génique dans des mélanocytes exposés à des UV étaient modulés. Par ailleurs, de nombreuses publications scientifiques démontrent que les SESNs interviennent également pour limiter les dégâts induits par le stress oxydatif.

Les substances cosmétiques les plus utilisées en tant que dépigmentants sont plus particulièrement l'acide ascorbique et ses dérivés, dont le glucoside d'ascorbyle, ainsi que certains extraits de plantes (notamment de réglisse).

Il subsiste néanmoins le besoin d'un nouvel agent blanchissant de la peau humaine ayant une bonne efficacité et qui soit d'origine naturelle, et bien toléré.

A cet égard, la Demanderesse a maintenant trouvé qu'un extrait alcoolique de parties aériennes d'*Anthyllis vulneraria,* obtenu par un procédé particulier, présente, par son action de diminution de la synthèse de mélanine des mélanocytes, des activités intéressantes vis-à-vis de la pigmentation et de l'unification du teint de la peau. En effet, comme démontré en exemples, l'extrait alcoolique de parties aériennes d*'Anthyllis vulneraria* selon l'invention présente des propriétés cosmétiques intéressantes : il permet une action blanchissante de la peau, notamment par induction de l'expression de SESN2 en mélanocytes et kératinocytes. Son action sur induction de l'expression de SESN2 peut également permettre la prévention et/ou l'atténuation d'altérations de la peau dues au vieillissement.

L'invention concerne donc, selon un premier aspect, un extrait alcoolique de parties aériennes d*'Anthyllis vulneraria,* susceptible d'être obtenu par un procédé comprenant les étapes suivantes :
a) extraction des parties aériennes d*'Anthyllis vulneraria,* avec au moins un solvant alcoolique ;
b) décantation du mélange obtenu en a) pendant au moins 10h ;
c) filtration du mélange décanté obtenu en b) ; et
d) élimination du solvant du filtrat obtenu, puis dilution finale dans un autre solvant alcoolique.

Un tel extrait est ainsi appelé, dans la présente demande, extrait selon l'invention.

L'invention se rapporte également à un procédé d'extraction de parties aériennes d*'Anthyllis vulneraria,* comprenant les étapes suivantes :
a) extraction des parties aériennes d'*Anthyllis vulneraria,* avec au moins un solvant alcoolique ;
b) décantation du mélange obtenu en a) pendant au moins 10h ;
c) filtration du mélange décanté obtenu en b) ; et
d) élimination du solvant du filtrat obtenu, puis dilution finale dans un autre solvant alcoolique.

L'invention se rapporte également à une composition cosmétique ou dermatologique comprenant, dans un véhicule cosmétiquement ou pharmaceutiquement acceptable, un extrait alcoolique de parties aériennes d*'Anthyllis vulneraria* selon l'invention. Par véhicule cosmétiquement ou pharmaceutiquement acceptable, on entend un milieu compatible avec la peau, les muqueuses et les phanères. De préférence, la composition cosmétique ou dermatologique selon l'invention est adaptée à une application par voie topique.

Le procédé d'obtention de l'extrait selon l'invention comprend ainsi les étapes suivantes :
a) extraction des parties aériennes d*'Anthyllis vulneraria,* avec au moins un solvant alcoolique ;
b) décantation du mélange obtenu en a) pendant au moins 10h ;
c) filtration du mélange décanté obtenu en b) ; et
d) élimination du solvant du filtrat obtenu, puis dilution finale dans un autre solvant alcoolique.

La matière première mise en œuvre est constituée des parties aériennes d*'Anthyllis vulneraria.*

L'anthyllide vulnéraire (*Anthyllis vulneraria*) appelée aussi Vulnéraire, Thé des Alpes, Trèfle des sables, est une plante herbacée éphémère annuelle ou pérenne, de la famille des Fabaceae. C'est une plante basse de 20 à 60 cm de hauteur s'étendant en couvre-sol. Elle présente des rosettes denses de feuilles vert foncé, couvertes de poils soyeux. Ses inflorescences sont jaunes oranges, groupées en capitules, calice laineux, apparaissant de mai à août, jusqu'en septembre en altitude. C'est une espèce héliophile qui pousse dans les endroits ensoleillés, souvent en bord de mer et dans les prairies sèches, les pelouses de montagne et les zones boisées. Elle présente une large gamme altitudinale, jusqu'à 3200m, une forte tolérance au froid et à la sécheresse.

Avec une croissance rapide, et un système racinaire profondément ancré, il assure la stabilisation rapide de la couche arable. C'est une plante pionnière, elle peut utiliser des substrats pauvres en matière organique et coloniser les terres meurtries. Comme toutes les Fabaceae, sa capacité à fixer l'azote atmosphérique peut rapidement enrichir le sol, favorisant ainsi la croissance des autres plantes.

On compte de nombreuses sous-espèces telles que
- *Anthyllis vulneraria* L. subsp. *alpestris* (Kit.) Asch. & Gr.
- *Anthyllis vulneraria* L. subsp. *bocsii*
- *Anthyllis vulneraria* L. subsp. *maritima*
- *Anthyllis vulneraria* L. subsp. *rubrifolia*
- *Anthyllis vulneraria* L. subsp. *vulneraria*

Les parties aériennes d*'Anthyllis vulneraria* utilisées selon l'invention sont typiquement choisies parmi les fleurs, les feuilles, les tiges et leurs mélanges. De préférence, les parties aériennes utilisées sont un mélange de fleurs, feuilles et tiges d*'Anthyllis vulneraria.* De préférence, ces parties aériennes sont préalablement séchées, puis broyées ou réduites en morceaux de manière usuelle, pour, de préférence, se présenter sous la forme d'une poudre de taille inférieure à 2 cm.

Dans l'étape a), les parties aériennes sont soumises à une extraction par un ou plusieurs solvants alcooliques, par exemple choisis parmi :
- les monoalcools en C₁-C₄, tels que par exemple le méthanol, l'éthanol ou l'isopropanol ; et
- les diols, tels que par exemple le propylène glycol, le 1,3-propanediol ou le dipropylène glycol.

De préférence, le solvant alcoolique est un monoalcool comprenant de 2 à 4 atomes de carbone, plus préférentiellement l'éthanol.

L'extraction est généralement réalisée en immergeant ou en agitant doucement les parties aériennes dans un ou plusieurs des solvants cités ci-dessus à des températures allant, par exemple, de la température ambiante à 80°C, pendant une durée d'environ 30 minutes à 8 h. De préférence, l'extraction de l'étape a) est réalisée pendant une durée comprise entre 2 et 6 heures, à une température comprise entre 40°C et 60°C.

En particulier, le rapport pondéral d*'Anthyllis vulneraria*/solvant alcoolique compris entre 1/1 et 1/20, et de préférence est de 1/10.

Selon un mode particulier de réalisation, l'étape a) d'extraction est réalisée deux fois.

Le mélange obtenu à l'étape a) est ensuite décanté pendant au moins 10h : c'est l'étape b). De préférence, l'incubation de l'étape b) est réalisée pendant une durée comprise entre 12h et 30h, à une température comprise entre 2°C et 30°C. Plus préférentiellement, la décantation est effectuée pendant une nuit, de préférence pendant 12h à 15h, à une température comprise entre 4 et 20°C.

Le mélange obtenu à l'étape a) peut être tamisé avant l'étape b) de décantation afin d'éliminer les résidus végétaux. Avantageusement, le tamisage est effectué sur un tamis de taille de maille comprise entre 50 µm et 100 µm.

Le mélange décanté obtenu à l'issue de l'étape b) est alors filtré afin d'éliminer les substances insolubles : c'est l'étape c). De préférence, la filtration de l'extrait obtenu en b) est effectuée sur une membrane de 4 µm. On obtient ainsi un filtrat liquide, de préférence transparent.

Enfin, le solvant présent dans le filtrat liquide est éliminé, puis le reste de filtrat est dilué dans un autre solvant alcoolique : c'est l'étape d). Le solvant alcoolique utilisé dans l'étape d) est appelé « autre solvant alcoolique », car il est différent du solvant alcoolique utilisé dans l'étape a). Tenant compte de cette limitation, le solvant alcoolique est typiquement choisi dans le même groupe que celui de l'étape a), i.e. parmi les monoalcools en C₁-C₄ et les diols.

De préférence, l'élimination du solvant de l'étape d) se fait par évaporation. De préférence, la dilution finale est réalisée dans un diol, de préférence du 1,3-propanediol. En particulier, la dilution finale peut être réalisée mélange d'eau et de 1,3-propanediol. Par exemple, un mélange de solvants constitué de 80% de 1, 3-propanediol, 10% d'eau est ajouté à 10% de poudre (poids/poids). Pour faciliter la solubilisation du filtrat, l'éthanol peut être ajouté et éliminé par évaporation.

De préférence, entre les étapes c) et d), une étape de décoloration du filtrat obtenu en c) est ajoutée. La décoloration peut se faire par adsorption des pigments tels que les chlorophylles et xanthophylles présents dans le filtrat sur charbon actif. Cette étape de décoloration peut être suivie d'une ou plusieurs étapes de filtration du filtrat décoloré obtenu, notamment de filtration sur média filtrant jusqu'à un seuil de filtration de 1 µm.

Selon un mode particulier de réalisation, le procédé selon l'invention met, en outre en œuvre une étape e) de filtration, en particulier jusqu'à un seuil de filtration 4 µm.

Préférentiellement, l'extrait alcoolique de parties aériennes d*'Anthyllis vulneraria* selon l'invention est susceptible d'être obtenu par un procédé comprenant les étapes suivantes :
a) une première extraction d'un mélange de fleurs, de feuilles et de tiges d*'Anthyllis vulneraria,* préalablement séchées et broyées, avec de l'éthanol, à une température comprise entre 50°C et 70°C pendant 2h à 5h puis tamisage entre 50 µm et 100µm;
a') une seconde extraction du produit obtenu à l'étape a) avec de l'éthanol, à une température comprise entre 50°C et 70°C pendant 2h à 5h, puis tamisage à 100µm, b) décantation du mélange des filtrats obtenu en a) et a') pendant au moins 12h à une température comprise entre 2°C et 30°C ;
c) filtration du mélange décanté obtenu en b), pour obtenir un filtrat ;
   - décoloration du filtrat obtenu en c) par adsorption sur charbon actif ; puis
   - filtration du filtrat décoloré jusqu'à un seuil de filtration de 1 µm ; et
d) élimination de l'éthanol du filtrat obtenu par évaporation, puis dilution finale dans du 1,3-propanediol, de préférence dans un mélange eau/1,3-propanediol,
e) filtration jusqu'à un seuil de filtration de 4 µm.

Avantageusement, l'extrait mis en œuvre selon l'invention est de couleur claire.

Egalement, ledit extrait se présente sous une forme suffisamment concentrée pour pouvoir être utilisé sans entraîner les problèmes de formulation habituellement rencontrés aux concentrations nécessaires pour obtenir une activité dans les compositions cosmétiques ou dermatologiques sous forme d'émulsion, et sans présenter une couleur foncée, contrairement aux extraits végétaux obtenus par des procédés usuels, lorsqu'ils sont sous forme concentrée.

De ce fait, l'extrait selon l'invention peut être utilisé directement pour la préparation d'une composition cosmétique ou dermatologique.

Selon un aspect ultérieur, l'invention concerne l'utilisation cosmétique d'un extrait alcoolique de parties aériennes d*'Anthyllis vulneraria* selon l'invention, en tant qu'agent dépigmentant, et/ou agent blanchissant et/ou agent éclaircissant, pour unifier le teint, et/ou pour corriger les imperfections pigmentaires.

En effet, de manière avantageuse, on a trouvé que l'extrait alcoolique de parties aériennes d*'Anthyllis vulneraria* selon l'invention possédait plusieurs activités d'intérêt vis-à-vis de mécanismes physiologiques permettant de diminuer la synthèse de mélanine.

L'invention concerne donc, plus particulièrement, l'utilisation cosmétique d'un extrait alcoolique de parties aériennes d'*Anthyllis vulneraria* selon l'invention en tant qu'agent inhibiteur de la synthèse de mélanine.

On a également trouvé que, de manière avantageuse, l'extrait alcoolique de parties aériennes d*'Anthyllis vulneraria* selon l'invention présentait une activité avantageuse à l'égard de l'expression de SESN2 dans les mélanocytes et/ou les kératinocytes.

L'invention concerne également l'utilisation d'un extrait alcoolique de parties aériennes d*'Anthyllis vulneraria* selon l'invention en tant qu'agent activateur de l'expression de SESN2 dans les mélanocytes et/ou les kératinocytes.

L'invention concerne aussi, plus particulièrement, l'utilisation cosmétique d'un extrait alcoolique de parties aériennes d*'Anthyllis vulneraria* selon l'invention pour la dépigmentation et/ou le blanchiment de la peau, notamment par son action inhibitrice de la synthèse de mélanine.

L'invention concerne encore l'utilisation cosmétique d'un extrait alcoolique de parties aériennes d*'Anthyllis vulneraria* selon l'invention en tant qu'antioxydant et/ou agent améliorant la microcirculation cutanée, et/ou pour la prévention et/ou l'atténuation d'altérations de la peau dues au vieillissement.

L'invention concerne également, selon un aspect ultérieur, une composition cosmétique ou dermatologique comprenant, dans un véhicule cosmétiquement ou pharmaceutiquement acceptable, un extrait alcoolique de parties aériennes d*'Anthyllis vulneraria* selon l'invention. De préférence, ledit extrait est présent dans la composition cosmétique ou dermatologique à raison de 0,001 à 10% en poids total de la composition, en particulier à raison de 0,01 à 10%, de préférence de 0,1 à 10% en poids total de la composition. Ladite composition cosmétique ou dermatologique peut notamment, être adaptée à une application par voie topique.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique peut également comprendre au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Notamment, ladite composition peut contenir :
- Un ou plusieurs agent(s) émollient(s) ou humectant(s), qui peuvent être choisi(s) par exemple parmi la glycérine, les glycols, les silicones hydrosolubles tels que celui vendu sous la dénomination KF6011 (Shin Etsu) et le Jojoba hydrosoluble, tel que celui vendu sous la dénomination Resplanta jojoba (Res pharma).
   Ledit agent émollient ou humectant peut être présent dans la composition à une teneur de l'ordre de 0 à 30%, de préférence 2 à 10% en poids, par rapport au poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghénanes, pectine), d'origine microbienne (xanthane), les argiles (laponite), les matériaux identifiés par les noms INCI "ammonium acryloyldiméthyltaurate/vp copolymer" et "ammonium acryloyldiméthyl-taurate/beheneth-25 méthacrylate copolymer" (tels que par exemple ceux vendus sous les dénominations Aristoflex AVC et HMB par Clariant).
   Ledit agent gélifiant et/ou épaississant peut être présent dans la composition à une teneur de l'ordre de 0 à 10% en poids, par rapport au poids total de la composition.
- Un ou plusieurs agent(s) tensioactif(s), de préférence non ionique, présent dans une teneur de l'ordre de 0 à 8%, de préférence 0,5 à 3% en poids, par rapport au poids total de la composition.
- Un ou plusieurs corps gras liquide(s) à température ambiante, communément dénommé(s) huiles(s), volatil(s) ou non volatile(s), hydrocarboné(s) ou siliconé(s), linéaire(s), cyclique(s) ou ramifié(s), par exemple, l'isododécane, le cyclopentadimethylsiloxane, les diméthicones, l'isononanoate d'isononyle ou le pentaerythrityl tetraisostéarate, de préférence à raison de 0 à environ 10%, de préférence 0,5 à 5% en poids, par rapport au poids total de la composition.
- Un ou plusieurs agent(s) actif(s), d'origine naturelle ou synthétique, ayant une activité biologique, par exemple choisis parmi les vitamines, les oligo-éléments, l'allantoïne, les protéines végétales, les extraits végétaux, les agents hydratants, les agents antiâges, les antioxydants, les agents favorisant l'éclat et des mélanges de ceux-ci. En particulier, l'agent actif est choisi parmi une eau de fruit de vanilla planifolia, le niacinamide, l'acide hyaluronique et ses dérivés, un extrait de levure et des mélanges de ceux-ci.
- Un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0 à environ 2% en poids, par rapport au poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention est illustrée de manière non limitative par les exemples ci-dessous.

### Exemple 1: Préparation d'un extrait alcoolique de parties aériennes d'Anthyllis vulneraria selon l'invention

Un extrait alcoolique de parties aériennes d*'Anthyllis vulneraria* selon l'invention est préparé par un procédé comprenant les étapes suivantes:
a) une première extraction d'un mélange de fleurs, de feuilles et de tiges d*'Anthyllis vulneraria,* préalablement séchées et broyées, avec de l'éthanol, à une température de 60°C pendant au moins 2h et tamisage de 50 µm à 100 µm pour obtenir le filtrat 1;
a') une seconde extraction du produit obtenu à l'étape a) sur les drêches retenues lors du tamisage de l'étape a), avec de l'éthanol, à une température comprise de 60°C pendant au moins 2h, puis le mélange est tamisé entre 50 µm et 100µm afin d'éliminer les résidus végétaux pour obtenir le filtrat 2
b) décantation du mélange des filtrats 1 et 2 obtenu en a) et a') pendant une nuit (au moins 12h) à une température comprise entre 2°C et 25 °C ;
c) filtration du mélange décanté obtenu en b) jusqu'au seuil de 1µm, pour obtenir un filtrat ;
   - décoloration du filtrat obtenu en c) par adsorption sur charbon actif ; puis
   - filtration du filtrat décoloré jusqu'au seuil de 1 µm ; et
d) élimination de l'éthanol du filtrat obtenu par évaporation, puis dilution finale dans un mélange eau/1,3-propanediol 12%/88%,
e) filtration jusqu'à un seuil de filtration 4 µm.

### Exemple 2: Test de cytotoxicité de l'extrait alcoolique d'Anthyllis vulneraria dans les kératinocytes humains normaux

### Protocole:

Des kératinocytes épidermiques humains normaux (PromoCell) provenant de donneurs juvéniles ont été cultivés dans des plaques 96 puits jusqu'à une confluence proche de 75%. Les cellules ont ensuite été incubées avec différentes concentrations de l'extrait alcoolique *d'Anthyllis vulneraria,* chaque concentration en triplicats, pendant 48h. La cytotoxicité a été évaluée à l'aide du Cell Titer96 Aqueous One Solution Cell Proliferation Assay (Promega), basé sur la capacité des cellules viables à réduire les sels de tétrazolium incolores/jaunes en dérivé formazan coloré intensément brun. Les cellules ont été incubées avec le tétrazolium à 37°C pendant 30 minutes et l'absorbance du formazan formé a été lue à 490 nm.

### Résultats:

La cytotoxicité de l'extrait alcoolique *d'Anthyllis vulneraria* a été évaluée à différentes concentrations comprises entre 0,1 et 0,0125% (tableau 1 ci-dessous).

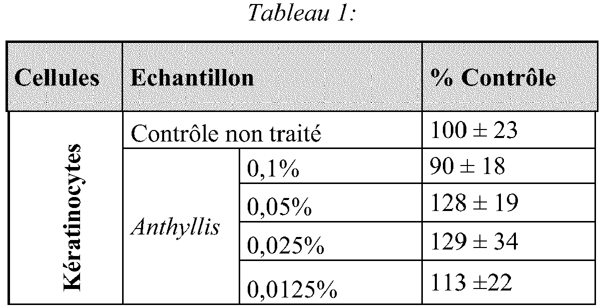

L'extrait alcoolique d*'Anthyllis vulneraria* n'est pas toxique pour les kératinocytes à toutes les concentrations testées.

Ces concentrations seront donc utilisées dans les expériences suivantes sur les deux types cellulaires (kératinocytes et mélanocytes).

### Exemple 3: Evaluation de l'effet de l'extrait alcoolique d'Anthyllis vulneraria sur l'expression génique de SESN2

### Protocole:

Les kératinocytes et les mélanocytes ont été cultivés dans une plaque à 6 puits jusqu'à une confluence proche de 75% avant d'être traités pendant 24h aux concentrations de l'extrait alcoolique d*'Anthyllis vulneraria* suivantes :
- 0.1%
- 0.05%

L'ARN total a été extrait en utilisant le kit RNeasy (Qiagen, cat# 74182) conformément au protocole du fabricant, quantifié par spectrophotométrie (Thermo Fisher Scientific, Multiskan GO) et retro-transcrit en cDNA en utilisant du iScript Reverse Transcription Supermix kit (Biorad, cat# 1708840).

Le cDNA a ensuite été utilisé en PCR quantitative en temps réel (qRT-PCR, Biorad, CFX96) pour l'analyse de l'expression génique de SESN2 en utilisant les sondes Taqman appropriées (Thermo Fisher Scientific) et des sondes correspondant à des gènes de ménages pour la normalisation.

Les résultats sont exprimés en "fold change" d'expression de SESN2 de la condition traitée par rapport à la condition contrôle.

### Résultats:

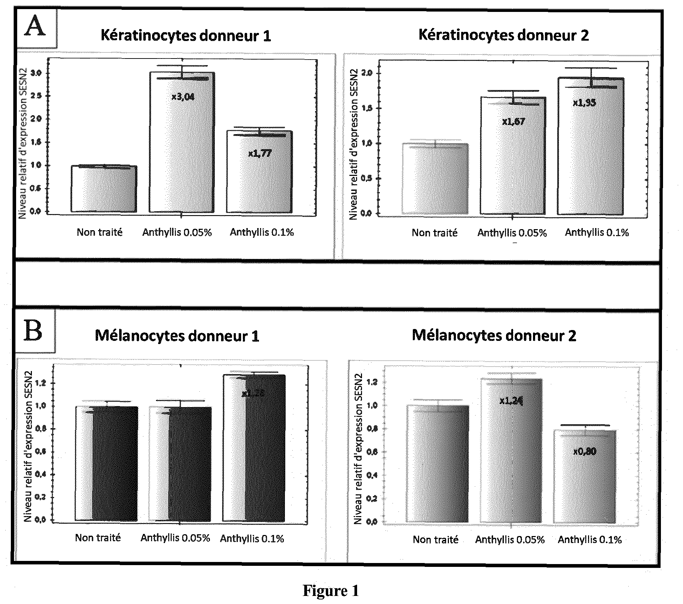

Dans les 2 types cellulaires, nous constatons une induction de l'expression de SESN2 (donneur et dose dépendante, comme illustré à la figure 1), d'approximativement x3.04 max en kératinocytes et x1.28 max en mélanocytes.

Il est donc possible de conclure que l'extrait alcoolique *d'Anthyllis vulneraria* est un ingrédient efficace pour réguler la réponse au stress, remodeler la matrice extracellulaire, photovieillissement, pigmentation et donc dans la homéostasie de la peau.

### Exemple 4: Détermination de l'effet de l'extrait alcoolique d'Anthyllis vulneraria sur la synthèse de mélanine dans des mélanocytes humains en culture

### Protocole:

Des mélanocytes ont été cultivés en plaque 6 puits jusqu'à une confluence de 50% max..

Les cellules sont incubées avec différentes concentrations de l'extrait alcoolique *d'Anthyllis vulneraria* (concentrations non toxiques allant de 0,1 à 0,0125% testées à l'exemple 1), en duplicats, pendant 5 jours avec un renouvellement de traitement d'actif tous les 2 jours. Les cellules sont ensuite lysées et la mélanine solubilisée en NaOH 1 M à 60°C pendant 1h.

Pour l'extraction de la mélanine intracellulaire, les cellules ont été lysées dans du NaOH 1M, centrifugées à 12 000 tours par minute pendant 5 minutes, et l'absorbance des surnageants clairs a été mesurée à 490 nm. La teneur en mélanine a été normalisée par rapport aux protéines totales par puits à 595 nm (Biorad Protein Assay, Biorad).

L'acide kojique, dont l'action est largement documentée dans l'inhibition de la synthèse de mélanine, a été utilisée comme contrôle positif.

### Résultats:

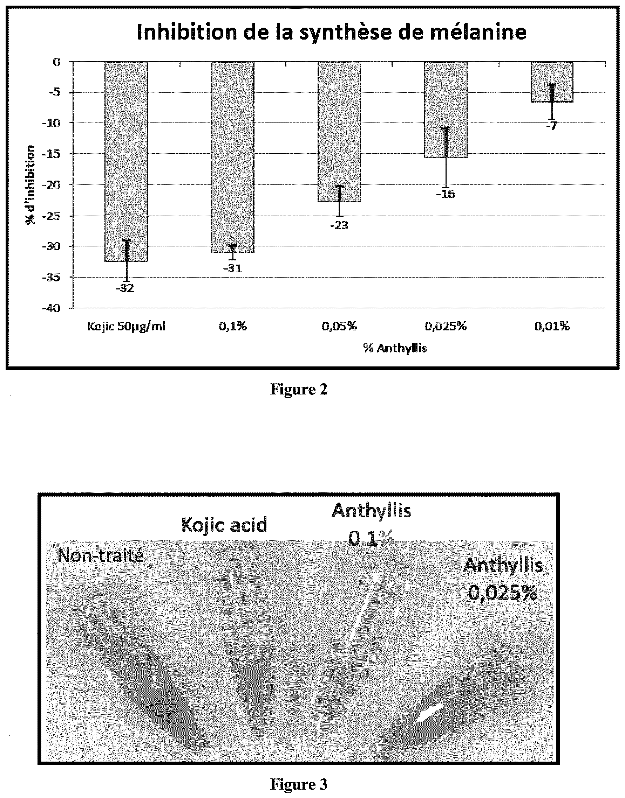

Les résultats, illustrés à la figure 2, sont représentés en % du témoin non traité qui est fixé à 100%.

La mélanine est le chromophore de la peau humaine synthétisé par les mélanocytes de l'épiderme et responsable principalement de la couleur de la peau. L'effet possible de l'extrait alcoolique d*'Anthyllis vulneraria* sur la pigmentation de la peau a été évalué par quantification chimique de mélanine dans les cellules traitées et non traitées.

L'extrait alcoolique d*'Anthyllis vulneraria* à la concentration de 0,1% inhibe de 31 ± 1.2% la teneur en mélanine des mélanocytes par rapport aux cellules non traitées.

Il ressort de ce test que l'extrait alcoolique d*'Anthyllis vulneraria* module la teneur en mélanine des mélanocytes cultivés et peut donc diminuer le niveau de pigmentation de la peau (donc agir comme agent dépigmentant).

Visuellement, l'apparence des lysats des cellules traitées avec l'Anthyllis est nettement plus clair que les cellules contrôle (comme illustré à figure 3).

### Exemple 5: compositions cosmétiques

### 5A - émulsion huile/eau crème gel

| **Nom INCI** | **(% W/W)** |
|---|---|
| Jojoba esters | 1-10 |
| Hydrogenated coconut oil | 1-10 |
| Moringa oil/hydrogenated moringa oil esters (FLORALIPIDS MORINGA BUTTER) | 1-10 |
| Butyrospermum parkii butter (LIPEX SHEASOFT) | 1-10 |
| Camellia kissi seed oil | 1-10 |
| Butyrospernum parkii butter extract (LIPEX SHEA TRIS) | 1-10 |
| Pentaerythrityl stearate/caprate/ caprylate/ adipate (SUPERMOL S-SO) | 0.5-5 |
| Cetyl ethylhexanoate | 1-5 |
| Octyle palmitate | 1-5 |
| Diisostearyl dimer dilinoleate (SCHERCEMOL DISD) | 1-10 |
| Octyldodecyl myristate | 1-5 |
| Hydro genated lecithin | 0.1-5 |
| Cetearyl alcohol & cetearyl glucoside | 0.1-7 |
| Glyceryl stearate & PEG-100 stearate | 0.1-5 |
| CARBOMER | 0.01-5 |
| BIOSACCHARIDE GUM-1 | 1-10 |
| Methyl methacrylate crosspolymer (MAKIBEADS 150) | 0.1-10 |
| Sodium hyaluronate | 0.01-3 |
| Glycerin | 1-30 |
| Polyquaternium-51 | 1-10 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| Extrait alcoolique d*'Anthyllis vulneraria* | 0.01-10 |
| Ascorbyl glucoside | 0.001-5 |
| Glycols (Caprylyl Glycol and/or Pentylene Glycol and/or Butylene Glycol and/or propanediol) | 0,1-10 |
| Water | Qs 100 |

### 5b - émulsion huile/eau crème

| **Nom INCI** | **(% w/w)** |
|---|---|
| Behenyl alcohol | 1-5 |
| Cetyl alcohol | 0.1-5 |
| Phenyl trimethicone | 1-5 |
| Dimethicone & Dimethicone/Vinyl Dimethicone Crosspolymer | 1-30 |
| Ectoin | 0.1-5 |
| PPG-2 myristyl ether propionate | 1-10 |
| Nanofine Titanium Dioxide | 1-20 |
| Zinc Dioxide | 1-20 |
| Diethylamino Hydroxybenzoyl Hexyl Benzoate (Uvinul A+) | 1-5 |
| Bis-Ethylhexyloxyphenol Methoxyphenyl Triazine (Tinosorb S) | 1-5 |
| (Tinosorb M) | 1-5 |
| Ethyl hexyl Methoxycinnamate | 1-7.5 |
| Polysilicone -11 | 1-5 |
| Silica | 1-5 |
| Polymethylsilsesquioxane | 1-5 |
| C20-22 alkyl phosphate & C20-22 alcohols | 0.5-5 |
| Glyceryl stearate & PEG-100 stearate | 0.5-5 |
| sodium acrylate / sodium acryloyldimethyltaurate copolymer | 0.1-5 |
| Hydrogenated starch hydrolysate & maltooligosyl glucoside | 0.1-10 |
| Xanthan Gum | 0,01-2 |
| Agar | 0.1-5 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| Extrait alcoolique d*'Anthyllis vulneraria* | 0.01-10 |
| Ascorbyl glucoside | 0.001-5 |
| Water | Qs 100 |

Ces compositions peuvent être appliquées tous les jours, matin et/ou soir, sur la peau.

## Revendications

1. Extrait alcoolique de parties aériennes *d'Anthyllis vulneraria,* susceptible d'être obtenu par un procédé comprenant les étapes suivantes :
a) extraction des parties aériennes d'Anthyllis vulneraria, avec au moins un solvant alcoolique ;
b) décantation du mélange obtenu en a) pendant au moins 10h ;
c) filtration du mélange décanté obtenu en b) ; et
d) élimination du solvant du filtrat obtenu, puis dilution finale dans un autre solvant alcoolique.

2. Extrait alcoolique de parties aériennes *d'Anthyllis vulneraria* selon la revendication 1, **caractérisé en ce que** le solvant alcoolique de l'étape a) est un monoalcool comprenant de 1 à 4 atomes de carbone, de préférence l'éthanol.

3. Extrait alcoolique de parties aériennes d*'Anthyllis vulneraria* selon la revendication 1 ou 2, **caractérisé en ce que** l'extraction de l'étape a) est réalisée pendant une durée comprise entre 2 et 6 heures, à une température comprise entre 40°C et 60°C.

4. Extrait alcoolique de parties aériennes d*'Anthyllis vulneraria* selon l'une des revendications 1 à 3, **caractérisé en ce que** les parties aériennes sont choisies parmi les fleurs, les feuilles, les tiges et leurs mélanges.

5. Extrait alcoolique de parties aériennes d*'Anthyllis vulneraria* selon l'une des revendications 1 à 4, **caractérisé en ce que** l'étape a) est réalisée deux fois.

6. Extrait alcoolique de parties aériennes d*'Anthyllis vulneraria* selon l'une des revendications 1 à 5, **caractérisé en ce que** la décantation de l'étape b) est réalisée pendant une durée comprise entre 12h et 30h, à une température comprise entre 2°C et 30°C.

7. Extrait alcoolique de parties aériennes d*'Anthyllis vulneraria* selon l'une des revendications 1 à 6, **caractérisé en ce que** le mélange obtenu à l'étape a) est tamisé avant l'étape b) de décantation, de préférence sur un tamis de taille de maille comprise entre 50 µm et 100 µm.

8. Extrait alcoolique de parties aériennes d*'Anthyllis vulneraria* selon l'une des revendications 1 à 7, **caractérisé en ce que**, entre les étapes c) et d), est ajoutée une étape de décoloration du filtrat obtenu en c), de préférence par adsorption sur charbon actif, suivie d'une étape ou plusieurs étapes de filtration du filtrat décoloré obtenu.

9. Extrait alcoolique de parties aériennes d*'Anthyllis vulneraria* selon l'une des revendications 1 à 8, **caractérisé en ce que** l'élimination de l'étape d) se fait par évaporation, puis la dilution finale est réalisée dans du 1,3-propanediol

10. Extrait alcoolique de parties aériennes d*'Anthyllis vulneraria* selon l'une des revendications 1 à 8, **caractérisé en ce que** le procédé met en œuvre une étape e) de filtration.

11. Extrait alcoolique de parties aériennes *d'Anthyllis vulneraria* selon l'une des revendications 1 à 10, **caractérisé en ce qu'**il est susceptible d'être obtenu par un procédé comprenant les étapes suivantes :
a) une première extraction d'un mélange de fleurs, de feuilles et de tiges d*'Anthyllis vulneraria,* préalablement séchées et broyées, avec de l'éthanol, à une température comprise entre 50°C et 70°C pendant 2h à 5h ;
a') une seconde extraction du produit obtenu à l'étape a) avec de l'éthanol, à une température comprise entre 50°C et 70°C pendant 2h à 5h, puis tamisage entre 50 µm et 100µm,
b) décantation du mélange des filtrats obtenus en a) et a') pendant au moins 12h à une température comprise entre 2°C et 30°C ;
c) filtration du mélange décanté obtenu en b), pour obtenir un filtrat ;
- décoloration du filtrat obtenu en c) par adsorption sur charbon actif ; puis
- filtration du filtrat décoloré jusqu'à un seuil de 1 µm ; et
d) élimination de l'éthanol du filtrat obtenu par évaporation, puis dilution finale dans du 1,3-propanediol, de préférence dans un mélange eau/1,3-propanediol,
e) filtration jusqu'à un seuil de filtration 4 µm.

12. Procédé d'extraction de parties aériennes d*'Anthyllis vulneraria,* comprenant les étapes suivantes :
a) extraction des parties aériennes d*'Anthyllis vulneraria,* avec au moins un solvant alcoolique ;
b) décantation du mélange obtenu en a) pendant au moins 10h ;
c) filtration du mélange décanté obtenu en b) ; et
d) élimination du solvant du filtrat obtenu, puis dilution finale dans un autre solvant alcoolique.

13. Utilisation non-thérapeutique d'un extrait alcoolique de parties aériennes d*'Anthyllis vulneraria* selon l'une quelconque des revendications 1 à 10 pour unifier le teint, et/ou pour corriger les imperfections pigmentaires.

14. Utilisation non-thérapeutique d'un extrait alcoolique de parties aériennes d*'Anthyllis vulneraria* selon l'une quelconque des revendications 1 à 10 comme agent dépigmentant et/ou agent blanchissant et/ou agent éclaircissant et/ou inhibiteur de la synthèse de mélanine.

15. Composition cosmétique ou dermatologique comprenant, dans un véhicule cosmétiquement ou pharmaceutiquement acceptable, un extrait alcoolique de parties aériennes d*'Anthyllis vulneraria* selon l'une quelconque des revendications 1 à 10.

16. Composition cosmétique ou dermatologique selon la revendication 15, **caractérisée en ce qu'**elle est adaptée à une application par voie topique.

## Patentansprüche

1. Alkoholischer Extrakt aus oberirdischen Teilen von *Anthyllis vulneraria,* welcher durch ein Verfahren erhalten werden kann, das die folgenden Schritte umfasst:
a) Extrahieren der oberirdischen Teile von Anthyllis vulneraria mit wenigstens einem alkoholischen Lösungsmittel;
b) Dekantieren der in a) erhaltenen Mischung während eines Zeitraums von wenigstens 10 Stunden;
c) Filtrieren der in b) erhaltenen dekantierten Mischung; und
d) Entfernen des Lösungsmittels aus dem erhaltenen Filtrat, dann abschließende Verdünnung in einem anderen alkoholischen Lösungsmittel.

2. Alkoholischer Extrakt aus oberirdischen Teilen von *Anthyllis vulneraria* nach Anspruch 1, **dadurch gekennzeichnet, dass** das alkoholische Lösungsmittel in Schritt a) ein Monoalkohol mit 1 bis 4 Kohlenstoffatomen, bevorzugt Ethanol, ist.

3. Alkoholischer Extrakt aus oberirdischen Teilen von *Anthyllis vulneraria* nach Anspruch 1 oder 2, **dadurch gekennzeichnet, dass** die Extraktion in Schritt a) über einen Zeitraum von 2 bis 6 Stunden bei einer Temperatur zwischen 40 °C und 60 °C durchgeführt wird.

4. Alkoholischer Extrakt aus oberirdischen Teilen von *Anthyllis vulneraria* nach einem der Ansprüche 1 bis 3, **dadurch gekennzeichnet, dass** die oberirdischen Teile gewählt sind aus Blüten, Blättern, Stängeln und deren Mischungen.

5. Alkoholischer Extrakt aus oberirdischen Teilen von *Anthyllis vulneraria* nach einem der Ansprüche 1 bis 4, **dadurch gekennzeichnet, dass** Schritt a) zweimal durchgeführt wird.

6. Alkoholischer Extrakt aus oberirdischen Teilen von *Anthyllis vulneraria* nach einem der Ansprüche 1 bis 5, **dadurch gekennzeichnet, dass** das Dekantieren in Schritt b) über einen Zeitraum von 12 bis 30 Stunden bei einer Temperatur zwischen 2 °C und 30 °C durchgeführt wird.

7. Alkoholischer Extrakt aus oberirdischen Teilen von *Anthyllis vulneraria* nach einem der Ansprüche 1 bis 6, **dadurch gekennzeichnet, dass** die in Schritt a) erhaltene Mischung vor dem Dekantieren in Schritt b) gesiebt wird, bevorzugt durch ein Sieb mit einer Maschenweite zwischen 50 µm und 100 µm.

8. Alkoholischer Extrakt aus oberirdischen Teilen von *Anthyllis vulneraria* nach einem der Ansprüche 1 bis 7, **dadurch gekennzeichnet, dass** zwischen den Schritten c) und d) ein Schritt des Entfärbens des in c) erhaltenen Filtrats hinzugefügt ist, bevorzugt durch Adsorption auf Aktivkohle, gefolgt von einem oder mehreren Schritten der Filtration des erhaltenen entfärbten Filtrats.

9. Alkoholischer Extrakt aus oberirdischen Teilen von *Anthyllis vulneraria* nach einem der Ansprüche 1 bis 8, **dadurch gekennzeichnet, dass** das Entfernen in Schritt d) durch Verdampfen erfolgt und dann die abschließende Verdünnung in 1,3-Propandiol erfolgt.

10. Alkoholischer Extrakt aus oberirdischen Teilen von *Anthyllis vulneraria* nach einem der Ansprüche 1 bis 8, **dadurch gekennzeichnet, dass** das Verfahren einen Filtrationsschritt e) umfasst.

11. Alkoholischer Extrakt aus oberirdischen Teilen von *Anthyllis vulneraria* nach einem der Ansprüche 1 bis 10, **dadurch gekennzeichnet, dass** er durch ein Verfahren erhalten werden kann, welches die folgenden Schritte umfasst:
a) eine erste Extraktion einer Mischung aus Blüten, Blättern und Stängeln von *Anthyllis vulneraria,* die zuvor getrocknet und mit Ethanol bei einer Temperatur zwischen 50 °C und 70 °C über einen Zeitraum von 2 bis 5 Stunden gemahlen wurden;
a') eine zweite Extraktion des in Schritt a) erhaltenen Produkts mit Ethanol bei einer Temperatur zwischen 50 °C und 70 °C über einen Zeitraum von 2 bis 5 Stunden, dann Sieben zwischen 50 µm und 100 µm,
b) Dekantieren der Mischung der in a) und a') erhaltenen Filtrate über einen Zeitraum von wenigstens 12 Stunden bei einer Temperatur zwischen 2 °C und 30 °C;
c) Filtrieren der in b) erhaltenen dekantierten Mischung, um ein Filtrat zu erhalten;
- Entfärben des in c) erhaltenen Filtrats durch Adsorption auf Aktivkohle; dann
- Filtrieren des entfärbten Filtrats bis zu einem Schwellenwert von 1 µm; und
d) Entfernen des Ethanols aus dem durch Verdampfung erhaltenen Filtrat, dann abschließende Verdünnung in 1,3-Propandiol, bevorzugt in einer Wasser/1,3-Propandiolmischung;
e) Filtrieren bis zu einem Filtrationsschwellenwert von 4 µm.

12. Verfahren der Extraktion von oberirdischen Teilen von *Anthyllis vulneraria,* welches die folgenden Schritte umfasst:
a) Extrahieren der oberirdischen Teile von *Anthyllis vulneraria* mit wenigstens einem alkoholischen Lösungsmittel;
b) Dekantieren der in a) erhaltenen Mischung über einen Zeitraum von wenigstens 10 Stunden;
c) Filtrieren der in b) erhaltenen dekantierten Mischung; und
d) Entfernen des Lösungsmittels aus dem erhaltenen Filtrat, dann abschließende Verdünnung in einem anderen alkoholischen Lösungsmittel.

13. Nicht-therapeutische Verwendung eines alkoholischen Extrakts aus oberirdischen Teilen von *Anthyllis vulneraria* nach einem der Ansprüche 1 bis 10 zur Egalisierung des Teints und/oder zur Korrektur von Pigmentstörungen.

14. Nicht-therapeutische Verwendung eines alkoholischen Extrakts aus oberirdischen Teilen von *Anthyllis vulneraria* nach einem der Ansprüche 1 bis 10 als Depigmentierungsmittel und/oder Bleichmittel und/oder Aufhellungsmittel und/oder Melaninsyntheseinhibitor.

15. Kosmetische oder dermatologische Zusammensetzung, welche in einem kosmetisch oder pharmazeutisch akzeptablen Träger einen alkoholischen Extrakt aus oberirdischen Teilen von *Anthyllis vulneraria* nach einem der Ansprüche 1 bis 10 umfasst.

16. Kosmetische oder dermatologische Zusammensetzung nach Anspruch 15, **dadurch gekennzeichnet, dass** sie für eine topische Anwendung geeignet ist.

## Claims

1. Alcoholic extract of the aerial parts of *Anthyllis vulneraria,* able to be obtained by a method comprising the following steps:
a) extracting the aerial parts of Anthyllis vulneraria, with at least one alcoholic solvent;
b) decanting of the mixture obtained in a) for at least 10 h;
c) filtering of the decanted mixture obtained in b); and
d) removing the solvent from the filtrate obtained, then final diluting in another alcoholic solvent.

2. Alcoholic extract of the aerial parts of *Anthyllis vulneraria* according to claim 1, **characterised in that** the alcoholic solvent of the step a) is a monohydric alcohol comprising from 1 to 4 carbon atoms, preferably ethanol.

3. Alcoholic extract of the aerial parts of *Anthyllis vulneraria* according to claim 1 or 2, **characterised in that** the extracting of the step a) is carried out for a duration between 2 and 6 hours, at a temperature between 40°C and 60°C.

4. Alcoholic extract of the aerial parts of *Anthyllis vulneraria* according to one of claims 1 to 3, **characterised in that** the aerial parts are chosen from the flowers, the leaves, the stems and mixtures thereof.

5. Alcoholic extract of the aerial parts of *Anthyllis vulneraria* according to one of claims 1 to 4, **characterised in that** the step a) is carried out twice.

6. Alcoholic extract of the aerial parts of *Anthyllis vulneraria* according to one of claims 1 to 5, **characterised in that** the decanting of the step b) is carried out for a duration between 12 h and 30 h, at a temperature between 2°C and 30°C.

7. Alcoholic extract of the aerial parts of *Anthyllis vulneraria* according to one of claims 1 to 6, **characterised in that** the mixture obtained at the step a) is screened before the step b) of decanting, preferably over a screen with a mesh size between 50 µm and 100 µm.

8. Alcoholic extract of the aerial parts of *Anthyllis vulneraria* according to one of claims 1 to 7, **characterised in that**, between the steps c) and d), is added a step of discolouration of the filtrate obtained in c), preferably by adsorption on active charcoal, followed by a step or several steps of filtration of the discoloured filtrate obtained.

9. Alcoholic extract of the aerial parts of *Anthyllis vulneraria* according to one of claims 1 to 8, **characterised in that** the removing of the step d) is done via evaporation, then the final dilution is carried out in 1,3-propanediol

10. Alcoholic extract of the aerial parts of *Anthyllis vulneraria* according to one of claims 1 to 8, **characterised in that** the method implements a step e) of filtration.

11. Alcoholic extract of the aerial parts of *Anthyllis vulneraria* according to one of claims 1 to 10, **characterised in that** it is able to be obtained by a method comprising the following steps:
a) a first extracting of a mixture of flowers, leaves and stems of *Anthyllis vulneraria,* dried and ground beforehand, with ethanol, at a temperature between 50°C and 70°C for 2 h to 5 h;
a') a second extracting of the product obtained in the step a) with ethanol, at a temperature between 50°C and 70°C for 2 h to 5 h, the screening between 50 µm and 100 µm,
b) decanting of the mixture of filtrates obtained in a) and a') for at least 12 h at a temperature between 2°C and 30°C;
c) filtering of the decanted mixture obtained in b), in order to obtain a filtrate;
- discolouration of the filtrate obtained in c) by adsorption on activated charcoal; then
- filtering of the discoloured filtrate to a threshold of 1 µm; and
d) removing of the ethanol from the filtrate obtained by evaporation, then final diluting in 1,3-propanediol, preferably in a water/1,3-propanediol mixture,
e) filtering to a filtration threshold of 4 µm.

12. Method for extracting aerial parts of *Anthyllis vulneraria,* comprising the following steps:
a) extracting the aerial parts of *Anthyllis vulneraria,* with at least one alcoholic solvent;
b) decanting of the mixture obtained in a) for at least 10 h;
c) filtering of the decanted mixture obtained in b); and
d) removing the solvent from the filtrate obtained, then final diluting in another alcoholic solvent.

13. Non-therapeutic use of an alcoholic extract of aerial parts of *Anthyllis vulneraria* according to any of claims 1 to 10 to unify the skin complexion, and/or for correcting pigmentary imperfections.

14. Non-therapeutic use of an alcoholic extract of aerial parts of *Anthyllis vulneraria* according to any of claims 1 to 10 as a depigmentation agent and/or whitening agent and/or lightening agent and/or melanin synthesis inhibitor.

15. Cosmetic or dermatological composition comprising, in a cosmetically or pharmaceutically acceptable vehicle, an alcoholic extract of aerial parts of *Anthyllis vulneraria* according to any of claims 1 to 10.

16. Cosmetic or dermatological composition according to claim 15, **characterised in that** it is suitable for topical application.
